# EUROPÄISCHE PATENTSCHRIFT

(11) **EP 1 663 955 B1**
(45) Veröffentlichungstag und Bekanntmachung des Hinweises auf die Patenterteilung: **31.10.2007**
(21) Anmeldenummer: 04820833.4
(22) Anmeldetag: 02.08.2004
(51) Int. Cl.: C07C 249/04, C07C 249/08, C07C 251/38

(54) **COAMMOXIMIERUNG VON KETONEN**
COAMMOXIDATION OF KETONES
COAMMOXIMISATION DE CETONES

(30) Priorität: 25.09.2003 DE 10344469
(43) Veröffentlichungstag der Anmeldung: 07.06.2006
(73) Patentinhaber: Degussa GmbH, 40474 Düsseldorf (DE)
(72) Erfinder: HERWIG, Jürgen, 46569 Hünxe (DE); ROOS, Martin, 45721 Haltern am See (DE); OENBRINK, Georg, 48249 Dülmen (DE); GÜNZEL, Bernd, 45721 Haltern am See (DE); KUPPERT, Dirk, 45657 Recklinghausen (DE)
(86) Internationale Anmeldenummer: PCT/EP2004/051684
(87) Internationale Veröffentlichungsnummer: WO 2005/063691

(56) Entgegenhaltungen:
- US-A- 5 498 793
- US-A1- 2003 100 795
- US-A1- 2003 105 356

## Beschreibung

Die vorliegende Erfindung bezieht sich auf ein Verfahren zur Coammoximierung, also zur gleichzeitigen Ammoximierung von Ketonen insbesondere von cyclischen Ketonen wie Cyclododecanon und Cyclohexanon. Dabei wird unter Ammoximierung die Herstellung von Oximen aus Ketonen oder Aldehyden mit Wasserstoffperoxid und Ammoniak und mit einem Katalysator, der im wesentlichen aus Silicium, Titan und Sauerstoff besteht, wie beispielsweise Titansilicalit, verstanden.

Die Ammoximierung von Carbonylverbindungen ist in der Literatur oft beschrieben worden. So wird in EP 0 208 311 ein Verfahren zur Herstellung von Cyclohexanonoxim aus Cyclohexanon, Ammoniak, Wasserstoffperoxid und einem Titansilicalit als Katalysator beschrieben. In dieser Schrift wird nur Cyclohexanon als Carbonylverbindung eingesetzt.

In EP 0496 385 wird ein mehrstufiges Verfahren zur Herstellung von Oximen aus Carbonylverbindungen durch Ammoximienmg in flüssiger Phase beschrieben. Es werden zwar verschiedene Carbonylverbindungen wie zum Beispiel Cyclohexanon, Acetophenon oder Cyclododecanon einzeln eingesetzt, aber es wird keinerlei Hinweis für den Einsatz von Mischungen von Carbonylverbindungen gegeben.

In EP 0 564 040 wird ein zweistufiges Verfahren zur Ammoximierung von Carbonylverbindungen in flüssiger Phase beschrieben. Auch hier wird jedoch nur die Ammoximierung einer carbonylischen Verbindung beansprucht, es gibt keinen Hinweis auf ggf. mögliche Mischungen von Carbonylverbindungen.

In DE-OS 2 111 792 schließlich wird ein Prozess zur Herstellung eines Cyclohexanonoxim/Cyclododecanonoxim-Gemisches beschrieben. Hier wird die Oximbildung nicht durch Ammoximierung, sondern durch klassische Oximbildung durch Umsetzen der Ketone mit Hydroxylamin in Form des Hydroxylaminsulfates erreicht Das Oximierungsverfahren wird in zwei Stufen durchgeführt, wobei die erste Reaktionsstufe bei einem pH von 3 - 4,5 und bei 80 - 90 °C und die zweite Reaktionsstufe bei einem pH von 5-6 und bei 90 - 110 °C ausgeführt wird Diese zweistufige Fahrweise wird als vorteilhaft beschrieben zur Minimierung der Hydroxylaminzersetzung durch Eisenionen, welche bei anderen Bedingungen zu hohen Verlusten des relativ teuren Hydroxylamineinsatzstoffes führen würde. Nachteile dieses Verfahrens sind u.a. der Einsatz des relativ teuren Hydroxylaminsulfates und die aufwändige Verfahrensweise in zwei Stufen sowie die genaue Einhaltung von pH-Bedingungen mit einhergehenden zusätzlichem Chemikalienverbrauch in Form von laufend zuzuführender Base z. B. von Ammoniak zur Neutralisation (Beispiel). Ebenso nachteilig ist der durch den Einsatz von Hydroxylaminsulfat verbundene Zwangsanfall an Sulfatsalz, das aufwändig entsorgt werden muss.

Die bekannten Ammoximierungsverfahren ausgehend von einfachen Grundstoffen Ammoniak und Wasserstoffperoxid beziehen sich lediglich auf den Einsatz von carbonylischen Einzelkomponenten, die nach einem für jede Komponente einzeln optimierten Verfahren hergestellt werden.

In der Literatur wird jedoch nirgendwo die Herstellung einer Mischung von verschiedenen Ketonoximen wie Cyclododecanonoxim und Cyclohexanonoxim nach einem modernen Ammoximierungsverfahren direkt ausgehend von den einfachen Grundstoffen Ammoniak und Wasserstoffperoxid beschrieben.

Ein solches Verfahren hätte jedoch den Vorteil, dass man für das mengenmäßig kleinere Produkt hier beispielsweise Cyclododecanonoxim den Scaleneffekt des mengenmäßig größeren Produktes Cyclohexanonoxim nutzen könnte, also ohne größere zusätzliche Investition z. B. in einer vorhandenen Caprolactamanlage neben Cyclohexanonoxim gleichzeitig auch Cyclododecanonoxim produzieren könnte.

Es bestand daher die Aufgabe, ein Verfahren zu finden, welches die Ammoximierung von Mischungen von Ketonen, besonders der technisch wichtigen Ketone Cyclohexanon und Cyclododecanon (CDON) nach einem modernen Ammoximierungsverfahren direkt ausgehend von den einfachen Grundstoffen Ammoniak und Wasserstoffperoxid und eines geeigneten Katalysators ermöglicht und das die Nachteile der bisher bekannten Verfahren vermeidet.

Gelöst werden diese sowie weitere nicht explizit genannten Aufgaben, die jedoch aus den hierin diskutierten Zusammenhängen ohne weiteres ableitbar oder erschließbar sind, durch ein Verfahren nach Anspruch 1. Zweckmäßige Ausformungen und Abwandlungen des erfindungsgemäßen Verfahrens werden in den auf Anspruch 1 rückbezogenen Unteransprüchen unter Schutz gestellt.

Dadurch, dass man zur Coammoximierung von mindestens zwei Ketonen, ein Verfahren anwendet, bei dem man ein Gemisch aus mindestens einem cyclischen Keton und mindestens einem weiteren Keton mit Ammoniak, Wasserstoffperoxid, einem Katalysator, der im wesentlichen aus Silicium, Titan und Sauerstoff besteht, in Gegenwart eines Lösungsmittels in einem Schritt zu einem entsprechenden Gemisch aus Ketonoximen umsetzt, gelingt es, die oben näher bezeichneten Nachteile des Standes der Technik zu überwinden.

Als Katalysator wird dabei bevorzugt Titansilicalit eingesetzt.

Für das erfindungsgemäße Verfahren kann ein Gemisch aus mindestens zwei Ketonen, beispielsweise ein Gemisch aus einem cyclischen und einem nicht cyclischen Keton eingesetzt werden. So können beispielsweise Mischungen aus Aceton und Cyclododecanon oder Acetophenon und Cyclododecanon, aber auch Mischungen anderer Ketone, die als Substrate für die Ammoximierung dienen können, verwendet werden.

Vorzugsweise wird aber ein Gemisch aus zwei oder mehreren cyclischen Ketonen ausgewählt aus der Gruppe cyclischer Ketone mit 5 bis 20 C-Atomen, insbesondere ausgewählt aus der Gruppe cyclischer Ketone mit 6 bis 12 C-Atomen, wie beispielsweise Cyclohexanon und Cyclooctanon, eingesetzt.

Erfindungsgemäß außerordentlich bevorzugt wird jedoch ein Gemisch aus Cyclohexanon und Cyclododecanon eingesetzt. In diesem Falle werden vorzugsweise Mischungen von Cyclohexanon und Cyclododecanon in einem Verhältnis von 10 : 1 bis 1 zu 10 Raumteilen, insbesondere von 5 : 1 bis 1 : 5 Raumteilen eingesetzt.

Die vorliegende Erfindung betrifft daher ein Verfahren zur Erzeugung von Mischungen von Oximen, bevorzugt cyclischen Oximen, insbesondere Mischungen von Cyclododecanonoxim und Cyclohexanonoxim aus den entsprechenden Ketonen, mit Wasserstoffperoxid und Ammoniak in Gegenwart eines Katalysatorsystems, wobei das Katalysatorsystem vorzugsweise aus mindestens zwei Komponenten besteht dergestalt, dass eine Komponente auf Basis von Titan, Silizium und Sauerstoff vorzugsweise in Form eines Titansilikalits, aufgebaut ist und optional eine zusätzliche Komponente aus mindestens einem Ammoniumsalz als Cokatalystor, besteht.

Weiterhin können in dem System noch mindestens ein Lösungsmittel oder mindestens ein Phasenvermittler wie z. B. ein oder mehrere Tenside enthalten sein.

Der Titan, Silizium und Sauerstoff enthaltende Katalysator kann als Feststoff sowohl kristallin als Pulver als auch als Formkörper verwendet werden. Wird der Katalysator als Formkörper eingesetzt, können weitere Komponenten, insbesondere neutrale oder saure anorganische oder organische Feststoffe vorhanden sein, wie Aluminiumoxid oder Siliziumoxid, die im Formkörper als Binder fungieren. Der Katalysator kann in der dem Fachmann geläufigen Weise in einem diskontinuierlichen oder in einem kontinuierlichen Reaktionssystem, z. B. einem kontinuierlich durchströmten Festbettreaktor, eingesetzt werden und die Coammoximierung entsprechend diskontinuierlich oder kontinuierlich betrieben werden.

Als homogener Cokatalysator für das erfindungsgemäße Verfahren können alle Ammoniumsalze eingesetzt werden, die in der Reaktionsmischung ausreichend löslich sind und deren Anionen sich nicht nachteilig auf den Verlauf der Reaktion auswirken. Nicht limitierende Beispiele sind Ammoniumsalze von starken Mineralsäuren, wie z. B. Ammoniumchlorid, Ammoniumsulfat, Ammoniumsilikat oder Ammoniumnitrat, sowie Ammoniumsalze von ein- oder mehrbasigen Carbonsäuren, wie z. B. Ammoniumformiat, Ammoniumacetat, Ammoniumpropionat, Ammoniumcitrat oder Ammoniumbenzoat. Die Menge des Ammoniumsalzes kann in weiten Grenzen gewählt werden. Bevorzugt wird das Ammoniumsalz in einer Konzentration von 0,001 mol/kg bis 1 mol/kg im Reaktionsgemisch eingesetzt. Das Ammoniumsalz wird vorzugsweise entweder direkt zur Reaktionsmischung gegeben oder dem zur Reaktion eingesetzten Wasserstoffperoxid zugegeben.

In einer weiteren Ausführungsform der Erfindung wird das als Cokatalysator verwendete Ammoniumsalz in der Reaktionsmischung aus einer Brönsted-Säure und dem zur Reaktion eingesetzten Ammoniak in situ erzeugt Nicht limitierende Beispiele für geeignete Brönsted-Säuren sind Mineralsäuren, wie z. B. Salzsäure, Schwefelsäure, Kieselsäure und Salpetersäure und ein- oder mehrbasige Carbonsäuren, wie z. B. Ameisensäure, Essigsäure, Propionsäure, Oxalsäure, Glutarsäure, Citronensäure oder Benzoesäure. Die Brönsted-Säure wird dabei vorzugsweise entweder direkt zur Reaktionsmischung gegeben oder dem zur Reaktion eingesetzten Wasserstoffperoxid zugegeben. Der Cokatalysator verbleibt nach der Reaktion in der wässrigen Phase.

Als Lösungsmittel können mit Wasser wenigstens teilweise mischbare Lösungsmittel, wie aliphatische Alkohole, z. B. Ethanol, Propanol, 1-Butanol, 2-Butanol, Iso-Butanol, tert-Butanol, in reiner Form oder als Mischungen verwendet werden. Hierbei bildet sich dann eine flüssige Phase. Es können weiterhin mit Wasser nicht mischbare Lösungsmittel, beispielsweise Aromaten wie Toluol oder, C₆ - C₁₂ -alicyclische Kohlenwasserstoffe verwendet werden, hierbei bilden sich dann zwei flüssige Phasen. Als nicht mit Wasser mischbare Lösungsmittel sind bevorzugt, Cyclooctan, Cyclododecan und Hexahydrocumol oder Mischungen daraus. Vorteil der letztgenannten Lösungsmittel ist, dass sie gegen Schwefelsäure resistent sind.

Zur Beschleunigung der Reaktion können - insbesondere bei Verwendung von mit Wasser nicht mischbaren Lösemitteln - Phasenvermittler eingesetzt werden.

Als Phasenvermittler können alle Tenside und Phasentransferkatalysatoren eingesetzt werden, soweit sie stabil sind, also nicht in situ oxidiert werden. Beispiele für solche Phasenvermittler sind Alkansulfonate, wie etwa Marlon PS 30 der Firma Sasol genannt. Weitere mögliche Phasenvermittler sind quarternäre Ammoniumsalze des Typs [NR¹R²R³R⁴]⁺ X⁻, wobei die Reste R¹ - R⁴ unabhängig voneinander aliphatische Kohlenwasserstoffreste von C₁ - C₂₀ sein können und X⁻ ein Anion wie z. B. Chlorid, Bromid, Iodid, Hydrogensulfat darstellen.

Beispielsweise können hier vorteilhaft Tetrabutylammoniumbromid, Tetrabutylammoniumchlorid, Tetraethylammoniumbromid, Tetraethylammoniumchlorid, Benzyltriethylammoniumchlorid, Benzyltrimethylammoniumchlorid, Methyltributylammoniumchlorid oder Methyltricaprylammoniumchlorid eingesetzt werden.

Als Phasenvermittler werden bevorzugt Alkansulfonate und/oder quarternäre Ammoniumsalze in einer Konzentration von 0,01 - 5 Gew.% bezogen auf die gesamte Reaktionsmischung eingesetzt

Wasserstoffperoxid wird als wässrige Lösung in handelsüblichen Konzentrationen, bevorzugt im Bereich von 10 % bis 70 %, insbesondere mindestens 30 %ig eingesetzt. Ammoniak wird entweder als konzentrierte, wässrige Lösung (mindestens 20 %ig) oder bevorzugt als reiner Ammoniak in gasförmiger oder kondensierter Form dem Reaktor zugeführt. Vorteile ergeben sich bei der Dosierung von gasförmigen bzw. kondensiertem Ammoniak und bei hoch konzentrierten Peroxidlösungen aus der geringeren Menge an eingeführtem Wasser, die bei der Aufarbeitung der Reaktionsmischung abgetrennt werden muss.

Die Reaktionstemperatur der Ammoximierung liegt im Bereich von 20 bis 150 °C, bevorzugt im Bereich von 50 bis 120 °C und besonders bevorzugt im Bereich von 60 bis 100 °C. Die Reaktion wird dabei entweder bei "Normaldruck", das heißt dem Dampfdruck des jeweiligen Lösemittels bei der Reaktionstemperatur oder bei einem Überdruck, vorzugsweise zwischen 1 und 10 bar durchgeführt. Der Überdruck kann mit Ammoniak oder einem Inertgas eingestellt werden. Wird der Reaktor verschlossen, steigt der Druck durch Bildung von gasförmigen Zersetzungsprodukten in Nebenreaktionen (vor allem Stickstoff und Sauerstoff) während der Reaktion an. Vorteilhaft ist es, den Reaktor isobar zu fahren, indem gasförmige Zersetzungsprodukte über einen leichten Abgasstrom beilspielsweise mit einem Blasenzähler im Labormaßstab oder einem technischen Druckregler kontrolliert entweichen können und gegebenenfalls verbrauchter Ammoniak nachdosiert wird

Bei der Ammoximierungsreaktion können Carbonylverbindungen und Wasserstoffperoxid jeweils diskontinuierlich oder kontinuierlich zudosiert werden. Da Zersetzungsreaktionen des H₂O₂ immer nebenbei auftreten, wird für einen vollständigen Keton-Umsatz ein Überschuss an Peroxidlösung benötigt, welcher durch geeignete Reaktionsführung und die erfindungsgemäßen Katalysatorsysteme minimiert werden kann. Bei den Versuchen hat es sich als vorteilhaft erwiesen, zu Beginn der Reaktion entweder die Carbonylverbindung vorzulegen oder sie in äquimolaren Mengen parallel zum Wasserstoffperoxid zu dosieren und den benötigten Überschuss an Peroxid nach beendeter Carbonylzugabe gemäß dem Verbrauch nachzudosieren.

Die auf diese Weise hergestellten Mischungen aus verschiedenen Ketonoximen, können im Anschluß auf bekannte Weise wie z. B. Kristallisation, ggf. Destillation etc. in ihre Einzelkomponenten aufgetrennt werden. Genauso gut ist es aber möglich, die Mischungen einer Folgereaktionsstufe zuzuführen und die dann dort anfallenden Produktgemische mit den genannten Trennmethoden aufzutrennen.

### Beispiel

In einen 1,5 1 Glas Reaktor wurden 450 g einer Lösung mit 12,5 Gew. % Cyclohexanon (574 mmol) und 12,5 Gew.-% Cyclododecanon (309 mmol), 241 g 25 %ig Ammoniaklösung (3,5 mol), 0,39 g Marlon PS30 und 7 g Ammoniumacetat eingefüllt Die Lösung wurde über ein externes Festbett, das 100 g eines Katalysator bestehend aus 80 % Titansilikalit (TS1) und 20 % Aluminiumoxid enthält mit einer Geschwindigkeit von 300 ml/min gepumpt.

Die Mischung wurde auf 85 °C erhitzt und dann wurden über 8 h 90 g 50 %ige Wasserstoffperoxidlösung (1,32 mol) zugepumpt. Nach dieser Zeit war der Umsatz von Cyclohexanon zu Cyclohexanonoxim vollständig und der Umsatz von Cyclododecanon zu Cyclododecanonoxim 96,5 %.

## Patentansprüche

1. Verfahren zur Coammoximierung von mindestens zwei Ketonen,
**dadurch gekennzeichnet,**
**dass** man ein Gemisch aus mindestens einem cyclischen Keton und mindestens einem weiteren Keton mit Ammoniak, Wasserstoffperoxid, einem Katalysator, der im wesentlichen aus Silicium, Titan und Sauerstoff besteht in einem Schritt zu einem entsprechenden Gemisch aus Ketonoximen umsetzt.

2. Verfahren nach Anspruch 1,
**dadurch gekennzeichnet,**
**dass** man ein Gemisch aus mindestens einem cyclischen Keton und mindestens einem weiteren Keton mit Ammoniak, Wasserstoffperoxid, einem Katalysator, der im wesentlichen aus Silicium, Titan und Sauerstoff besteht, in Gegenwart eines Lösungsmittels in einem Schritt zu einem entsprechenden Gemisch aus Ketonoximen umsetzt.

3. Verfahren nach Anspruch 1 oder 2,
**dadurch gekennzeichnet,**
**dass** zusätzlich mindestens ein Ammoniumsalz als Cokatalysator verwendet wird.

4. Verfahren nach einem der vorhergehenden Ansprüche,
**dadurch gekennzeichnet,**
**dass** man ein Gemisch aus zwei oder mehreren cyclischen Ketonen ausgewählt aus der Gruppe cyclischer Ketone mit 5 bis 20 C-Atomen einsetzt.

5. Verfahren nach Anspruch 4,
**dadurch gekennzeichnet,**
**dass** man ein Gemisch aus zwei oder mehreren cyclischen Ketonen ausgewählt aus der Gruppe cyclischer Ketone mit 6 bis 12 C-Atomen einsetzt.

6. Verfahren nach Anspruch 5,
**dadurch gekennzeichnet,**
**dass** als Gemisch aus cyclischen Ketonen ein Gemisch aus Cyclohexanon und Cyclododecanon eingesetzt wird.

7. Verfahren nach mindestens einem der vorhergehenden Ansprüche,
**dadurch gekennzeichnet,**
**dass** man Ammoniak in Konzentration von mindestens 20 % in Wasser oder reinen Ammoniak einsetzt.

8. Verfahren nach mindestens einem der vorhergehenden Ansprüche,
**dadurch gekennzeichnet,**
**dass** man wässriges Wasserstoffperoxid in Konzentration von 10 - 70 % einsetzt.

9. Verfahren nach mindestens einem der vorhergehenden Ansprüche,
**dadurch gekennzeichnet,**
**dass** als Katalysator Titansilicalit verwendet wird.

10. Verfahren nach einem der vorhergehenden Ansprüche,
**dadurch gekennzeichnet,**
**dass** als Cokatalysator ein Ammoniumsalz einer Mineralsäure und/oder einer Carbonsäure eingesetzt wird.

11. Verfahren nach einem der Ansprüche 2 bis 9,
**dadurch gekennzeichnet,**
**dass** der Cokatalysator in der Reaktionsmischung in situ aus einer Brönsted-Säure und Ammoniak erzeugt wird.

12. Verfahren nach einem der Ansprüche 2 bis 11,
**dadurch gekennzeichnet,**
**dass** das Ammoniumsalz in einer Konzentration von 0,001 bis 1 mol/kg im Reaktionsgemisch enthalten ist.

13. Verfahren nach einem der vorhergehenden Ansprüche,
**dadurch gekennzeichnet,**
**dass** als Lösungsmittel ein mit Wasser wenigstens teilweise mischbares Lösungsmittel oder ein mit Wasser nicht mischbares Lösungsmittel verwendet wird.

14. Verfahren nach Anspruch 13,
**dadurch gekennzeichnet,**
**dass** bei Verwendung eines mit Wasser nicht mischbaren Lösungsmittels noch zusätzlich ein Phasenvermittler eingesetzt wird.

15. Verfahren nach Anspruch 14,
**dadurch gekennzeichnet,**
**dass** als Phasenvermittler Alkansulfonate und/oder quarternäre Ammoniumsalze in einer Konzentration von 0,01 bis 5 Gew.%, bezogen auf die gesamte Reaktionsmischung, eingesetzt werden.

16. Verfahren nach einem der vorhergehenden Ansprüche,
**dadurch gekennzeichnet,**
**dass** die Reaktionstemperatur im Bereich von 20 bis 150 °C liegt.

17. Verfahren nach Anspruch 16,
**dadurch gekennzeichnet,**
**dass** die Reaktionstemperatur im Bereich von 50 bis 120 °C, bevorzugt im Bereich von 60 bis 100 °C liegt.

18. Verfahren nach einem der vorhergehenden Ansprüche,
**dadurch gekennzeichnet,**
**dass** die Coammoximierung in einem kontinuierlichen oder in einem diskontinuierlichen Reaktionssystem durchgeführt wird.

19. Verfahren nach einem der vorhergehenden Ansprüche,
**dadurch gekennzeichnet,**
**dass** die Reaktion bei einem Druck von 1 bis 10 bar durchgeführt wird.

## Claims

1. Process for the coammoximation of at least two ketones **characterized in that** it comprises reacting a mixture of at least one cyclic ketone and at least one further ketone with ammonia, hydrogen peroxide, a catalyst consisting essentially of silicon, titanium and oxygen, in the presence of a solvent in one step to give a corresponding mixture of ketone oximes.

2. Process according to Claim 1, **characterized in that** a mixture of at least one cyclic ketone and at least one further ketone is reacted with ammonia, hydrogen peroxide, a catalyst consisting essentially of silicon, titanium and oxygen, in the presence of a solvent in one step to give a corresponding mixture of ketone oximes.

3. Process according to Claim 1 or 2, **characterized in that**, in addition, at least one ammonium salt is used as cocatalyst.

4. Process according to one of the preceding claims, **characterized in that** use is made of a mixture of two or more cyclic ketones selected from the group consisting of cyclic ketones having 5 to 20 carbon atoms.

5. Process according to Claim 4, **characterized in that** use is made of a mixture of two or more cyclic ketones selected from the group consisting of cyclic ketones having 6 to 12 carbon atoms.

6. Process according to Claim 5, **characterized in that**, as mixture of cyclic ketones, use is made of a mixture of cyclohexanone and cyclododecanone.

7. Process according to at least one of the preceding claims, **characterized in that** use is made of ammonia at a concentration of at least 20% in water, or pure ammonia.

8. Process according to at least one of the preceding claims, **characterized in that** aqueous hydrogen peroxide is used at a concentration of 10-70%.

9. Process according to at least one of the preceding claims, **characterized in that** the catalyst used is titanium silicalite.

10. Process according to one of the preceding claims, **characterized in that**, as cocatalyst, use is made of an ammonium salt of a mineral acid and/or of a carboxylic acid.

11. Process according to one of Claims 2 to 9, **characterized in that** the cocatalyst is generated in the reaction mixture in situ from a Brönsted acid and ammonia.

12. Process according to one of Claims 2 to 11, **characterized in that** the ammonium salt is present in the reaction mixture at a concentration of 0.001 to 1 mol/kg.

13. Process according to one of the preceding claims, **characterized in that**, as solvent, use is made of an at least partially water-miscible solvent, or a water-immiscible solvent.

14. Process according to Claim 13, **characterized in that**, when a water-immiscible solvent is used, in addition an interphase contactor is used.

15. Process according to Claim 14, **characterized in that**, as interphase contactors, use is made of alkanesulfonates and/or quaternary ammonium salts at a concentration of 0.01 to 5% by weight, based on the total reaction mixture.

16. Process according to one of the preceding claims, **characterized in that** the reaction temperature is in the range from 20 to 150°C.

17. Process according to Claim 16, **characterized in that** the reaction temperature is in the range from 50 to 120°C, preferably in the range from 60 to 100°C.

18. Process according to one of the preceding claims, **characterized in that** the coammoximation is carried out in a continuous or in a batchwise reaction system.

19. Process according to one of the preceding claims, **characterized in that** the reaction is carried out at a pressure of 1 to 10 bar.

## Revendications

1. Procédé pour la co-ammoximation d'au moins deux cétones, **caractérisé,**
**en ce qu'**on transforme un mélange d'au moins une cétone cyclique et d'au moins une autre cétone avec de l'ammoniac, du peroxyde d'hydrogène, un catalyseur, qui est essentiellement constitué de silicium, de titane et d'oxygène, en une étape en un mélange correspondant de cétonoximes.

2. Procédé selon la revendication 1, **caractérisé**
**en ce qu'**on transforme un mélange d'au moins une cétone cyclique et d'au moins une autre cétone avec de l'ammoniac, du peroxyde d'hydrogène, un catalyseur, qui est essentiellement constitué de silicium, de titane et d'oxygène, en présence d'un solvant en une étape en un mélange correspondant de cétonoximes.

3. Procédé selon la revendication 1 ou 2, **caractérisé**
**en ce qu'**on utilise en outre au moins un sel d'ammonium comme cocatalyseur.

4. Procédé selon l'une quelconque des revendications précédentes, **caractérisé**
**en ce qu'**on utilise un mélange de deux cétones cycliques ou plus choisies dans le groupe des cétones cycliques comprenant 5 à 20 atomes de carbone.

5. Procédé selon la revendication 4, **caractérisé**
**en ce qu'**on utilise un mélange de deux cétones cycliques ou plus choisies dans le groupe des cétones cycliques comprenant 6 à 12 atomes de carbone.

6. Procédé selon la revendication 5, **caractérisé**
**en ce qu'**on utilise comme mélange de cétones cycliques un mélange de cyclohexanone et de cyclododécanone.

7. Procédé selon au moins l'une quelconque des revendications précédentes, **caractérisé**
**en ce qu'**on utilise l'ammoniaque en une concentration d'au moins 20% dans l'eau ou de l'ammoniac pur.

8. Procédé selon au moins l'une quelconque des revendications précédentes, **caractérisé**
**en ce qu'**on utilise du peroxyde d'hydrogène aqueux en une concentration de 10-70%.

9. Procédé selon au moins l'une quelconque des revendications précédentes, **caractérisé**
**en ce qu'**on utilise comme catalyseur du silicalite de titane.

10. Procédé selon l'une quelconque des revendications précédentes, **caractérisé**
**en ce qu'**on utilise comme cocatalyseur un sel d'ammonium d'un acide minéral et/ou d'un acide carboxylique.

11. Procédé selon l'une quelconque des revendications 2 à 9, **caractérisé**
**en ce que** le cocatalyseur est produit in situ dans le mélange réactionnel à partir d'un acide de Brônstedt et de l'ammoniaque.

12. Procédé selon l'une quelconque des revendications 2 à 11, **caractérisé**
**en ce que** le sel d'ammonium est contenu en une concentration de 0,001 à 1 mole/kg dans le mélange réactionnel.

13. Procédé selon l'une quelconque des revendications précédentes, **caractérisé**
**en ce qu'**on utilise comme solvant un solvant au moins partiellement miscible avec l'eau ou un solvant non miscible avec l'eau.

14. Procédé selon la revendication 13, **caractérisé**
**en ce qu'**on utilise, lors de l'utilisation d'un solvant non miscible avec l'eau, en outre encore un promoteur de phases.

15. Procédé selon la revendication 14, **caractérisé**
**en ce qu'**on utilise comme promoteur de phases des alcanesulfonates et/ou des sels d'ammonium quaternaire en une concentration de 0,01 à 5% en poids, par rapport à la totalité du mélange réactionnel.

16. Procédé selon l'une quelconque des revendications précédentes, **caractérisé**
**en ce que** la température de réaction se situe dans la plage de 20 à 150°C.

17. Procédé selon la revendication 16, **caractérisé**
**en ce que** la température de réaction se situe dans la plage de 50 à 120°C, de préférence dans la plage de 60 à 100°C.

18. Procédé selon l'une quelconque des revendications précédentes, **caractérisé**
**en ce que** la co-ammoximation est réalisée dans un système de réaction continu ou discontinu.

19. Procédé selon l'une quelconque des revendications précédentes, **caractérisé**
**en ce que** la réaction est réalisée à une pression à 1 jusqu'à 10 bars.
